Europäisches Patentamt

(19) European Patent Office

Office européen des brevets

(11) Numéro de publication : **0 348 272 B1**

(12)

# FASCICULE DE BREVET EUROPEEN

(45) Date de publication du fascicule du brevet :
28.08.91 Bulletin 91/35

(51) Int. Cl.⁵ : **A61B 17/58**

(21) Numéro de dépôt : **89401648.4**

(22) Date de dépôt : **13.06.89**

(54) Implant pour dispositif d'ostéosynthèse rachidienne, notamment en traumatologie.

(30) Priorité : 24.06.88 FR 8808538

(43) Date de publication de la demande :
27.12.89 Bulletin 89/52

(45) Mention de la délivrance du brevet :
28.08.91 Bulletin 91/35

(84) Etats contractants désignés :
CH DE ES GB IT LI

(56) Documents cités :
EP-A- 0 128 058
GB-A- 812 248
US-A- 2 992 669
US-A- 4 411 259

(73) Titulaire : **SOCIETE DE FABRICATION DE MATERIEL ORTHOPEDIQUE**
**60-62 rue Rothschild**
**F-62600 Berck/Mer (FR)**

(72) Inventeur : **Cotrel, Yves**
**24 BIS, avenue du Président Wilson**
**75016 Paris (FR)**

(74) Mandataire : **Martin, Jean-Paul et al**
**c/o CABINET LAVOIX 2, Place d'Estienne d'Orves**
**F-75441 Paris Cedex 09 (FR)**

## Description

La présente invention a pour objet un implant pour dispositif d'ostéosynthèse, notamment du rachis, du type comprenant une partie destinée à l'ancrage osseux et un corps de fixation sur une tige dans lequel est ménagé un canal débouchant sur une partie postérieure dudit corps, délimitant deux branches latérales et ouvert de part et d'autre du corps pour pouvoir recevoir la tige.

La partie d'ancrage osseux peut être par exemple une vis ou une lame convenablement réalisée.

Les corps des implants connus sont, soit fermés, soit ouverts postérieurement, soit ouverts latéralement.

Ces implants présentent les inconvénients suivants :

— le nombre d'éléments nécessaires pour l'instrumentation est très élevé, en raison de l'existence de trois types différents de corps ;

— les corps ouverts postérieurement entraînent l'obligation d'utiliser des bloqueurs intracanalaires, comme décrit par exemple dans le brevet français 2545350 (8307450), constituant des éléments d'instrumentation supplémentaires qui doivent être placés au préalable sur la tige ;

— il est relativement difficile d'introduire la tige dans les implants à corps fermés ;

— les implants à corps ouverts doivent être orientés de manière précise, ce qui constitue une sujétion gênante pour le praticien dans le cas de l'utilisation de vis ;

— la multiplicité et la complexité des éléments utilisés nécessitent la mise en oeuvre d'un matériel ancillaire important ;

— encombrement gênant ;

— la fabrication des éléments de ces implants est difficile et onéreuse, et leur ablation également difficile, en raison notamment de l'obligation d'exécuter des coupes.

L'invention a pour but de proposer un implant exempt de ces inconvénients.

Conformément à l'invention, l'implant comporte un bouchon fileté, adapté pour pouvoir être vissé dans un taraudage formé dans les parois intérieures des deux branches latérales en fermant le canal du côté dudit corps opposé à la partie destinée à l'ancrage osseux, et la face du bouchon orientée vers la tige est munie de moyens d'accrochage et de fixation du bouchon à celle-ci, qui peut ainsi être bloquée en translation et en rotation.

Le vissage du bouchon à l'intérieur des branches du corps grâce aux filets prévus sur les parois intérieures des branches assure à l'implant un encombrement minimum.

Suivant un mode de réalisation possible, les moyens d'accrochage comprennent une pointe centrale venue de matière avec le reste du manchon.

Ces moyens d'accrochage peuvent comprendre, en variante, une couronne périphérique faisant saillie de la face du bouchon, ou, de préférence, une combinaison de cette couronne périphérique et de la pointe centrale.

D'autres particularités et avantages de l'invention apparaîtront au cours de la description qui va suivre, faite en référence aux dessins annexés qui en illustrent un mode de réalisation à titre d'exemple non limitatif.

La figure 1 est une vue en perspective d'un mode de réalisation de l'implant selon l'invention, fixé sur une tige d'un dispositif d'ostéosynthèse.

La figure 2 est une vue en élévation de l'implant de la Fig. 1 dans la direction axiale de la tige, le bouchon étant représenté séparé du corps.

La figure 3 est une vue en coupe transversale de l'implant dans un plan perpendiculaire à la tige associée.

Les figures 4 et 5 sont des vues du bouchon de l'implant respectivement en coupe axiale et en perspective.

La figure 6 est une vue en plan de la face du bouchon tournée vers la tige.

La figure 7 est une vue dans un plan sagittal de vertèbres dont l'une est fracturée.

La figure 8 est une vue dans un plan frontal de la fracture de la Fig. 7 réduite par un dispositif d'ostéosynthèse pourvu d'implants selon l'invention.

La figure 9 est une vue dans le plan sagittal du dispositif de la Fig. 8.

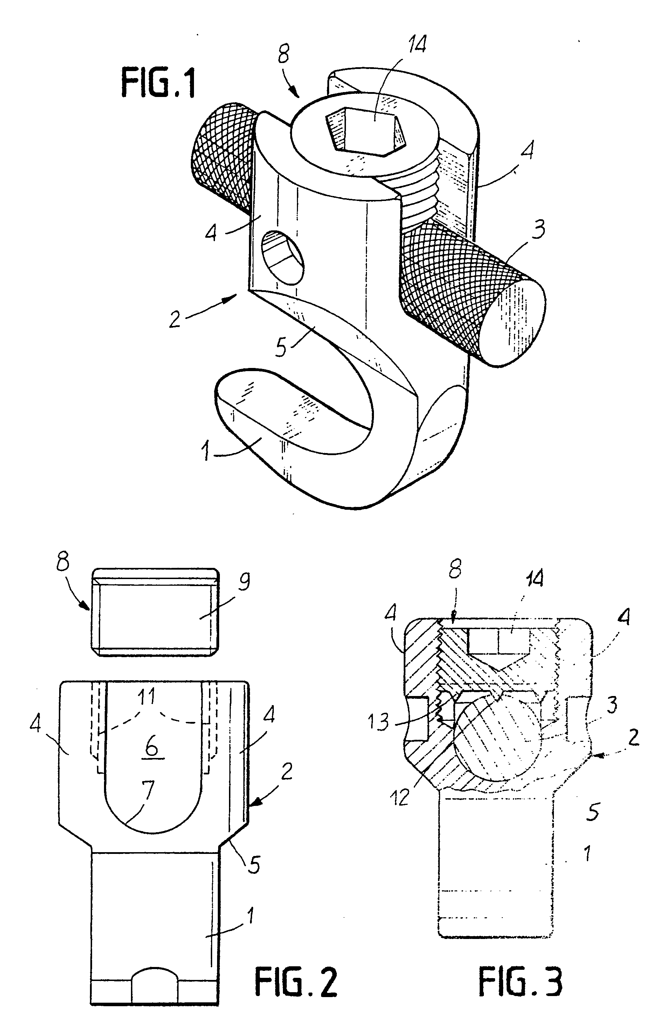

L'implant représenté aux Fig. 1 à 6 est destiné à faire partie d'un dispositif d'ostéosynthèse, notamment du rachis.

Il comprend une partie destinée à l'ancrage osseux, constituée dans cet exemple par une lame recourbée 1, connue en soi, et qui ne sera donc pas décrite, et un corps 2 de fixation sur une tige 3 dont la surface est lisse ou munie d'aspérités telles que des pointes de diamant.

Le corps 2 comporte, en partant de l'extrémité de la lame 1, deux branches latérales 4 reliées à la lame 1 par une partie conique 5 et qui délimitent entre elles un canal 6 dont un fond arrondi 7 est adapté pour pouvoir recevoir la tige 3, le canal 6 étant ouvert du côté opposé au fond arrondi 7. Le canal 6 est ouvert de part et d'autre des branches 4 pour pouvoir recevoir la tige 3 entre celles-ci.

L'implant comporte un bouchon mâle 8 pourvu d'un filetage 9 et adapté pour pouvoir être vissé dans un taraudage 11 formé dans les parois intérieures des deux branches 4, en fermant le canal 6 de ce côté.

Suivant une particularité complémentaire de l'invention, la face 8a du bouchon 8 orientée vers la tige 3 est munie de moyens d'accrochage et de fixation du bouchon 8 à celle-ci, qui peut ainsi être bloquée en translation et en rotation.

Dans le mode de réalisation décrit, ces moyens d'accrochage comprennent une pointe centrale 12 venue de matière avec le reste du bouchon 8, et une couronne périphérique 13 faisant saillie de la face 8a, de section triangulaire à sommet 13a de préférence arrondi, et venue de matière avec le reste du bouchon 8. La pointe 12 et la couronne périphérique 13 prennent appui sur la tige 3 en s'enfonçant dans celle-ci, lorsque le bouchon 8 est complètement vissé dans le taraudage 11.

La partie postérieure du bouchon 8, opposée à la couronne 13 et à la pointe 12, présente un trou profilé 14 pour permettre son vissage dans le taraudage 11 par un instrument approprié, ce profil 14 étant par exemple à six pans. Le taraudage 11 des deux flancs ou branches 4 du corps de l'implant et le filetage 9 du bouchon 8 peuvent être réalisés avec un pas en dents de scie, afin d'éviter l'écartement des deux flancs 4 en éliminant totalement la composante radiale de l'effort lors du vissage.

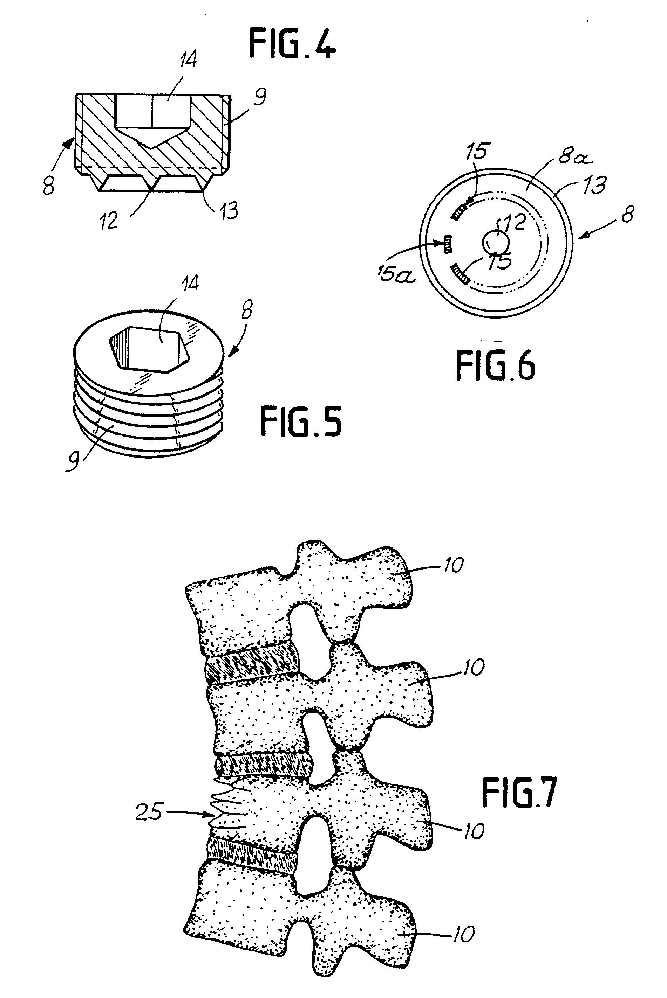

Le bouchon 8 présente, sur sa face 8a orientée vers la tige 3, des rugosités 15 (Fig. 6). Ces rugosités peuvent être avantageusement formées par un profil en dents de scie dont les parties obliques 15a sont orientées dans le sens du serrage du bouchon 8. Ce dernier est réalisé avantageusement par déformation à froid, par exemple par frappe à froid, ce qui lui confère une dureté supérieure à celle de la tige moletée 3.

La lame d'ancrage 1, le corps 2, le bouchon 8 sont réalisés en un même matériau biocompatible, par exemple l'acier selon la norme américaine 316L correspondant à la norme AFNOR AS 1 Z2 CND 17.13.

Le montage de l'implant qui vient d'être décrit est très simple : on introduit d'abord la tige 3 entre les branches ou berges 4 dans le canal 6 jusqu'à ce qu'elle vienne en appui dans le fond 7. Puis on visse partiellement le bouchon 8 dans le taraudage 11. On met en charge l'implant par détraction ou compression ou dérotation, on fixe ensuite l'implant par serrage du bouchon 8 par l'outil correspondant au profil du trou 14. Enfin on procède à la fixation terminale par vissage du bouchon 8 jusqu 'à ce que sa pointe centrale 12 et sa couronne 13 pénètrent dans la tige 3, cette pénétration étant rendue possible par le fait que la dureté du matériau constituant le bouchon 8 est supérieure à celle du matériau de la tige 3. De ce fait, la couronne 13 et la pointe 12 empêcheront tout desserrage de l'ensemble, la pointe 12 ainsi que deux extrémités diamétralement opposées de la couronne 13 étant en effet enfoncées dans la tige 3, de même que des rugosités 15.

Au terme de ces opérations, la tige 3 est bloquée à la fois en rotation et en translation. La pointe 12 et la couronne 13 assurent la sécurité de la fixation en agissant comme un frein d'écrou.

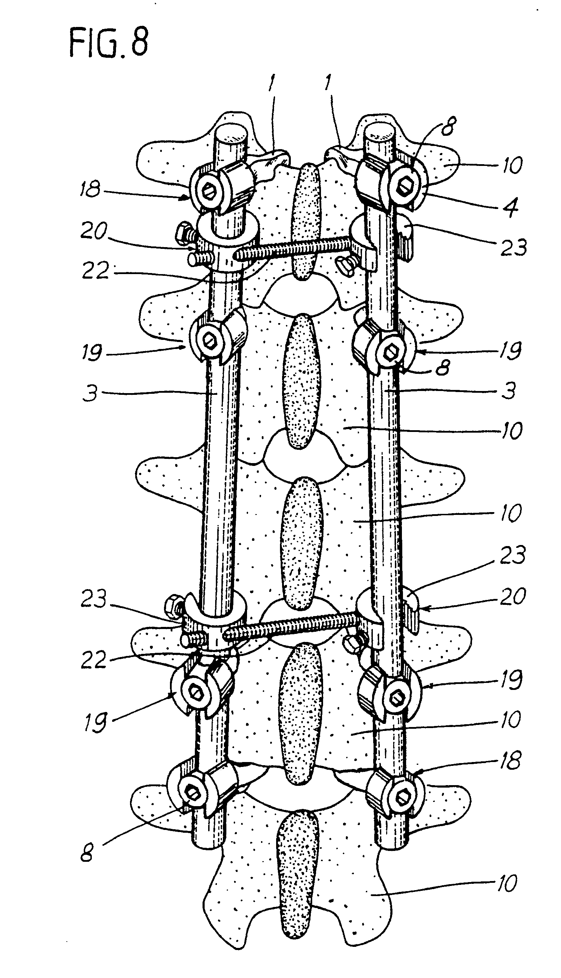

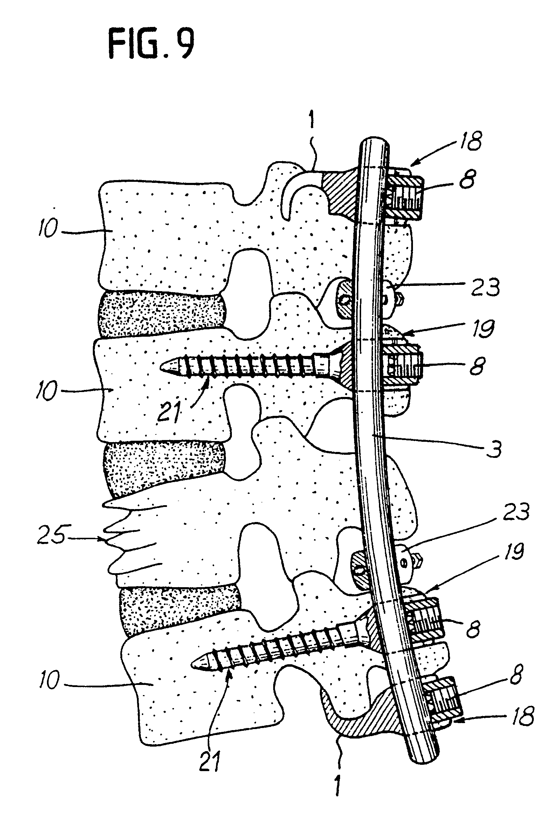

Les Fig. 8 et 9 montrent l'implantation d'une instrumentation d'ostéosynthèse sur des vertèbres 10 dont l'une présente une fracture 25 (Fig. 7). Cette instrumentation comprend deux dispositifs 20 de solidarisation et de traction transversale (DTT) à tiges transversales 22 et crochets 23 situés sur les tiges 3. L'instrumentation est complétée par plusieurs implants 18 et 19 semblables à celui décrit en référence aux Fig. 1-4, avec toutefois cette différence en ce qui concerne les implants 19 que dans ceux-ci la partie d'ancrage osseux n'est pas constituée par une lame recourbée 1, mais par une tige filetée 21, les implants 18 et 19 étant convenablement répartis le long des tiges 3.

L'implant selon l'invention présente les avantages suivants :

— possibilité d'introduction directe de la tige 3 postérieurement au corps 2 de l'implant sans nécessiter des pièces complémentaires, telles que des bloqueurs intracanalaires ;

— possibilité de réaliser la compression et la détraction (c'est-à-dire la mise en extension de la partie rachidienne ou osseuse) sans obligation d'une orientation précise de la tête dans le cas des implants à vis, alors qu'une orientation précise est au contraire imposée par la forme conique du système antérieur ;

— simplification considérable de l'instrumentation par la réduction des différents types de corps utilisés jusqu 'à présent à un seul type, ouverts postérieurement, et simplification corrélative du matériel ancillaire ;

— réduction considérable de l'encombrement extérieur de l'implant grâce au bouchon 8 vissé dans le taraudage intérieur 11 des flancs 4, le corps 2 de l'implant se présentant de plus de manière cylindrique ;

— élimination de tous risques de dégradation du taraudage 11 des flancs 4 par manipulation de l'implant, grâce au fait que ce taraudage est agencé intérieurement aux flancs 4 ;

— réduction du nombre de pièces nécessaires grâce à la pointe 12 et à la couronne 13 réalisées d'une seule pièce avec le bouchon 8, par rapport à un implant équipé d'un bouchon et d'une vis pointeau séparée. L'instrumentation nécessaire est donc réduite, et le montage de l'implant est simplifié pour le chirurgien.

— possibilité de remise en tension du dispositif (lorsque cela devient nécessaire par suite de la croissance d'un jeune sujet) ou d'ablation aisée, cette dernière étant surtout nécessaire en traumatologie. En effet, le démontage de l'implant peut être effectué très aisément d'abord par dévissage du bouchon 8 ;

— enfin facilité d'introduction de la tige dans l'implant, contrastant avec la difficulté actuelle d'introduction de cette tige dans un corps fermé.

En variante, le bouchon 8 peut être muni uniquement de sa pointe centrale 12, ou bien uniquement de sa couronne 13.

## Revendications

1. Implant pour dispositif d'ostéosynthèse, notamment du rachis, comprenant une partie (1) destinée à l'ancrage osseux et un corps (2) de fixation sur une tige (3), dans lequel le corps présente un canal (6) débouchant sur la partie du côté dudit corps opposé à la partie (1) destinée à l'ancrage osseux, ledit canal étant délimité par deux branches latérales (4) et ouvert de part et d'autre des branches latérales (4) pour pouvoir recevoir la tige (3) entre celles-ci, caractérisé en ce qu'il comporte un bouchon fileté (8), adapté pour pouvoir être vissé dans un taraudage (11) formé dans les parois intérieures des deux branches latérales (4) en fermant le canal (6) du côté dudit corps (2) opposé à la partie (1) destinée à l'ancrage osseux, et en ce que la face (8a) du bouchon (8) orientée vers la tige (3) est munie de moyens d'accrochage et de fixation du bouchon à celle-ci, qui peut ainsi être bloquée en translation et en rotation.

2. Implant selon la revendication 1, caractérisé en ce que lesdits moyens d'accrochage comprennent une pointe centrale (12) venue de matière avec le reste du bouchon (8).

3. Implant selon la revendication 1, caractérisé en ce que lesdits moyens d'accrochage comprennent une couronne périphérique (13) faisant saillie de la face (8a) du bouchon (8), de section triangulaire à sommet (13a) de préférence arrondi, et venue de matière avec le reste du bouchon.

4. Implant selon la revendication 1, caractérisé en ce que lesdits moyens d'accrochage comprennent une pointe centrale (12) et une couronne périphérique (13) saillant de la face (8a) du bouchon (8), et venues de matière avec ce dernier.

5. Implant selon l'une des revendications 1 à 4, caractérisé en ce que la partie d'ancrage (1), le corps (2) et le bouchon (8) sont réalisés en un même matériau biocompatible.

6. Implant selon l'une quelconque des revendications précédentes, caractérisé en ce que le taraudage intérieur (11) des deux branches (4) du corps (2) et le filetage (9) du bouchon (8) sont réalisés avec un pas en dents de scie afin d'éliminer une composante radiale lors du vissage.

7. Implant selon l'une quelconque des revendications précédentes, caractérisé en ce que le bouchon (8) présente des rugosités (15) sur sa face (8a) orientée vers la tige (3).

8. Implant selon la revendication 7, caractérisé en ce que les rugosités (15) sont formées par un profil en dents de scie dont la partie oblique (15a) est orientée dans le sens du serrage.

9. Implant selon l'une quelconque des revendications précédentes, caractérisé en ce que le bouchon (8) est réalisé par déformation à froid, par exemple frappe à froid, ce qui lui confère une dureté supérieure à celle de la tige moletée (3).

## Patentansprüche

1. Implantat für eine Vorrichtung zur Osteosynthese, insbesondere der Wirbelsäule, mit einem Abschnitt (1), der zur Knochenverankerung bestimmt ist, und einem Körper (2) zur Befestigung auf einer Stange (3), wobei der Körper einen Kanal (6) zeigt, der sich auf den Teil der Seite des Körpers gegenüber dem Abschnitt (1), der zur Knochenverankerung bestimmt ist, öffnet, wobei der Kanal durch zwei Seitenarme (4) begrenzt ist und beidseitig der Seitenarme (4) zum Aufnehmen der Stange (3) zwischen diesen geöffnet ist, **dadurch gekennzeichnet**, daß es einen Schraubverschluß (8) umfaßt, welcher so ausgelegt ist, daß er in ein Innengewinde (11) geschraubt werden kann, das in den Innenwänden der beiden Seitenarme (4) ausgebildet ist, um den Kanal (6) auf der Seite des Körpers gegenüber dem Abschnitt (1), der zur Knochenverankerung bestimmt ist, zu verschließen, und daß die auf die Stange (3) gerichtete Fläche (8a) des Verschlusses (8) mit einer Einrichtung zum Mitnehmen und zum Befestigen des Verschlusses an dieser versehen ist, der daher gegen Translation und Rotation gesperrt werden kann.

2. Implantat nach Anspruch 1, dadurch gekennzeichnet, daß die Mitnahmeeinrichtung eine mittige Spitze (12) aus demselben Material wie der übrige Verschluß (8) aufweist.

3. Implantat nach Anspruch 1 dadurch gekennzeichnet, daß die Mitnahmeeinrichtung eine periphere Krone (13), die aus der Fläche (8a) des Verschlusses hervorspringt, mit dreieckiger Schnittfläche und einer bevorzugt gerundeten Spitze (13a), aus demselben Material wie der übrige Verschluß (8) aufweist.

4. Implantat nach Anspruch 1, dadurch gekennzeichnet, daß die Mitnahmeeinrichtung eine mittige Spitze (12) und eine aus der Fläche (8a) des Verschlusses hervorspringende periphere Krone (13) aus demselben Material wie der übrige Verschluß (8) aufweist.

5. Implantat nach einem der Ansprüche 1 bis 4, dadurch gekennzeichnet, daß der Verankerungsabschnitt (1), der Körper (2) und der Verschluß (8) aus dem gleichen biokompatiblen Material gestaltet sind.

6. Implantat nach einem der vorangehenden Ansprüche, dadurch gekennzeichnet, daß das Innengewinde (11) der beiden Seitenarme (4) des Körpers (2) und das Gewinde (9) des Verschlusses (8) mit einer Sägezahnstufe gestaltet sind, um eine radiale Komponente während des Schraubens auszuschalten.

7. Implantat nach einem der vorangehenden Ansprüche, dadurch gekennzeichnet, daß der Verschluß (8) Unebenheiten (15) auf der zur Stange (3) hin gerichteten Fläche (8a) zeigt.

8. Implantat nach Anspruch 7, dadurch gekennzeichnet, daß die Unebenheiten (15) durch ein Sägezahnprofil gebildet sind, dessen schräger Bereich (15a) in Klemmrichtung ausgerichtet ist.

9. Implantat nach einem der vorangehenden Ansprüche, dadurch gekennzeichnet, daß der Verschluß (8) durch Kaltverformung, beispielsweise Kaltstauchen, gestaltet ist, was ihm eine Härte gibt, welche der der gerändelten Stange (3) überlegen ist.

## Claims

1. Implant for a device for osteosynthesis, in particular of the spine, comprising a part (1) intended for osseous anchoring and a body (2) for fixation on a rod (3), in which the body has a channel (6) opening out at that part of the side of the said body opposite the part (1) intended for osseous anchoring, the said channel being delimited by two lateral branches (4) and open on each side of the lateral branches (4) so as to be able to receive the rod (3) between the latter, characterised in that it comprises a threaded plug (8) designed to be able to be screwed in a tapping (11) formed in the inner walls of the two lateral branches (4), closing the channel (6) from that side of the said body (2) opposite the part (1) intended for osseous anchoring, and in that the face (8a) of the plug (8) oriented towards the rod (3) is provided with means for attachment and fixation of the plug to the rod, which can thus be locked in translation and in rotation.

2. Implant according to Claim 1, characterised in that the said means of attachment comprise a central point (12) made monobloc with the rest of the plug (8).

3. Implant according to Claim 1, characterised in that the said means of attachment comprise a peripheral collar (13) projecting from the face (8a) of the plug (8), of triangular cross-section with a preferably rounded vertex (13), and made monobloc with the rest of the plug.

4. Implant according to Claim 1, characterised in that the said means of attachment comprise a central point (12) and a peripheral collar (13) projecting from the face (8a) of the plug (8), these being made monobloc with the latter.

5. Implant according to one of Claims 1 to 4, characterised in that the anchoring part (1), the body (2) and the plug (8) are made of the same biocompatible material.

6. Implant according to any one of the preceding claims, characterised in that the inner tapping (11) of the two branches (4) of the body (2) and the thread (9) of the plug (8) are made with a sawtooth pitch in order to eliminate a radial component during screwing.

7. Implant according to any one of the preceding claims, characterised in that the plug (8) has roughened areas (15) on its face (8a) oriented towards the rod (3).

8. Implant according to Claim 7, characterised in that the roughened areas (15) are formed by a sawtooth profile whose oblique part (15a) is oriented in the direction of tightening.

9. Implant according to any one of the preceding claims, characterised in that the plug (8) is made by cold-deformation, for example cold-coining, which confers upon it a hardness greater than that of the knurled rod (3).

**FIG.1**

**FIG.2**

**FIG.3**

# FIG.4

# FIG.6

# FIG.5

# FIG.7

# FIG.8

# FIG. 9